# EUROPÄISCHE PATENTSCHRIFT

(11) **EP 2 553 156 B1**
(45) Veröffentlichungstag und Bekanntmachung des Hinweises auf die Patenterteilung: **08.12.2021**
(21) Anmeldenummer: 11751793.8
(22) Anmeldetag: 28.03.2011
(51) Int. Cl.: D04H 13/00, A61F 13/539, A61F 13/49, A61F 13/15, A61F 13/532, B32B 5/02, B32B 5/04, B32B 5/26, B32B 7/12, A61F 13/534, A61F 13/53

(54) **HOCHFLEXIBLES ABSORBIERENDES LAMINAT UND VERFAHREN ZU DESSEN HERSTELLUNG**
HIGHLY FLEXIBLE ABSORBENT LAMINATE AND METHOD FOR PRODUCING SAME
STRATIFIÉ ABSORBANT TRÈS SOUPLE ET SON PROCÉDÉ DE PRODUCTION

(30) Priorität: 29.03.2010 DE 102010013288
(43) Veröffentlichungstag der Anmeldung: 06.02.2013
(73) Patentinhaber: Evonik Superabsorber GmbH, 45128 Essen (DE)
(72) Erfinder: FENSKE, Wilfried, 64 683 Einhausen (DE)
(74) Vertreter: Evonik Patent Association
(86) Internationale Anmeldenummer: PCT/DE2011/000338
(87) Internationale Veröffentlichungsnummer: WO 2011/141009

(56) Entgegenhaltungen:
- WO-A1-2004/011046
- JP-A- 2002 192 641
- US-A- 5 366 452
- US-A1- 2006 206 073
- US-A1- 2008 156 418
- None

## Beschreibung

Die Erfindung betrifft ein Verfahren zur Herstellung eines hochflexiblen, absorbierenden Laminats nach Anspruch 1.

### In diesem Zusammenhang werden auch offenbart

flächige absorbierende Materialien, die mindestens in einer Koordinatenrichtung ausdehnbar sind und der Volumenzunahme durch Aufnahme der zu absorbierenden Flüssigkeit Raum schaffen.

Insbesondere werden absorbierende Materialien im Zusammenhang mit Einwegartikeln wie z.B. Babywindeln, Inkontinenzprodukten und der Damenhygiene, oder im Bereich absorbierender Artikel der Verpackungs- und Lebensmitteltechnik nachgefragt. Traditionell wird diese Aufgabe durch geeignete Kombinationsprodukte aus Zellstoff, superabsorbierende Granulaten oder -fasern und flüssigkeitsverteilenden Schichten aus synthetischen Vliesstoffen, Zellstoff oder Baumwolle erfüllt.

Aus ökonomischer und ökologischer Sicht sowie der Nachhaltigkeit sollte der Verbrauch an Rohstoffen und Verpackungsmaterialien sowie der Aufwand der für Rohstoffe für Produktion und Vertrieb benötigt wird, so gering wie möglich gehalten werden. Für die Herstellung von Hygieneartikeln bedeutet dies in erster Linie die Reduzierung des Primärenergieverbrauch, des Zellstoffs, der Transportvolumen, Verpackungsfolien und -kartonage und Abfallvolumen.

Im Bereich der Hygieneartikel sind sowohl aus der Literatur, als auch aus der industriellen Produktion vielfältige Projekte bekannt, die den teilweisen oder vollständigen Ersatz der Saugfähigkeit von Zellulose- bzw. Zellstoff durch wasserabsorbierende Polymere (Superabsorber) Granulate erfolgen soll. So ist es technisch, ökonomisch und ökologisch wünschenswert, beispielsweise moderne Babywindeln oder Inkontinenzartikel für iErwachsene komplett auf zellstofffreie Saugkissen umzustellen, um den Einfluss der Verwendung von Einwegartikeln auf die Klimaentwicklung so gering wie möglich zu halten.

### STAND DER TECHNIK

Es werden zwei grundsätzlich unterschiedliche Ansätze zur Reduzierung, bzw. Eliminierung des Zellstoffs aus den absorbierenden Kissen von Babywindeln, Inkontinenzprodukten und Damenhygieneartikeln verfolgt: Im ersten Ansatz wird auf den Ersatz des Zellstoffs durch dehn- oder quellfähige Thermoplaste als Binder zwischen den Granulaten des Superabsorbers abgezielt, welche beim Aufquellen des Superabsorbers die Haftung erhalten, die Quellbewegung mitmachen und die Integrität des Substrats auch in feuchtem Zustand gewährleisten.

Im anderen Fall handelt es sich um die Einlagerung des Superabsorbers zwischen zwei oder mehreren Trägerschichten in diskreten Sektionen, wobei die Volumenvergrößerung des Superabsorbers durch dessen Quellung durch Flüssigkeitsaufnahme alternativ durch Elastizität von einer oder mehrerer Trägerschichten, Raffung oder Dehnung einer oder mehrerer Trägerschichten während der Einbringung des Superabsorbers in das Laminat oder durch geeignete Verbindung der einzelnen Trägerschichten zueinander erfolgt, dass ein gezieltes, lokales Aufplatzen dieser Verbindung aufgrund des Berstdrucks durch die Quellung des Superabsorbers ermöglicht wird, ohne das der Superabsorber vollständig aus den Trägerschichten austritt. )

So ist aus der EP 724 418 die Herstellung eines Laminats beschrieben, das aus zwei äußeren Lagen besteht, von denen wenigstens eine Lage hydrophil ist und die mittels eines wassersensiblen Haftklebers miteinander verleimt sind, so dass in isolierten unverleimten Sektionen punktuell Superabsorber eingelagert werden kann, der im gequollenen Zustand gezielt die Verleimung aufbricht und die für das Quellvolumen erforderliche Volumenerhöhung des Laminats erreicht. Hierbei ist es von Nachteil, dass im gequollenen Zustand nur eine minimale Integrität des Laminats erreicht werden kann.

Aus der US 20020102392 ist ebenfalls ein Verfahren zur Herstellung eines saugfähigen Laminats mit eingelagerten Sektionen von Supeabsorbern und elastischen Eigenschaften bekannt. Hier befinden sich zwischen zwei äußere Lagen, von denen eine in Längsrichtung mittels einer Profilwalze gerafft wird, und quer zur Produktionsrichtung Sektionen von Superabsorbern über ein Vakuumsystem positioniert werden und ein in Längsrichtung dehnbares Laminat erzielt, wobei die Elastizität und Raffung durch Verwendung elastischer Folien oder Vliesstoffe erhöht werden kann.

Aus der US20020115969 ist ein weiteres Verfahren zur Herstellung eines Laminats bekannt. Hier wird die längskontinuierlichen Herstellung eines Laminats mit Einzelbahnen von Superabsorbern beschrieben, die mittels Heißleims zwischen zwei äußere Lagen dergestalt eingebracht sind, das quer zur Produktionsrichtung jeweils Streifen von superabsorberbedeckten und superabsorberfreien Bereichen vorhanden sind.

Die Herstellung eines Laminats aus zwei äußeren Lagen mit punktuell eingelagerten Sektionen von Superabsorbern wird in WO 2004071539 und WO 2004071363 beschrieben. Mittels einer texturierten Vakuumwalze wird eine erste äußere Schicht zur Ausbildung von Vertiefungen gebracht, diese mit Superabsorbern und Faserstoffen gefüllt und mit der zweiten äußeren Schicht verbunden. Vergleichbare Produkte werden seit längerem auch als Unterlagen im OP-Bereich und in der Krankenversorgung verwendet.

US 2008156418 A1 offenbart ein Verfahren zur Herstellung eines elastischen Laminats aus zwei textilen Materialbahnen. Es handelt sich um einen endlosen Rolle-zu-Rolle Prozess. Das elastische Laminat ist zur Herstellung von Hygieneartikeln wie Baby-Windeln gedacht.

US 2006206073 A1 beschreibt einen Absorberkern, wie er in Hygieneartikeln wie Baby-Windeln eingebaut wird. Der Absorberkern umfasst ein gerafftes elastisches, textiles Material das mit Superabsobern gefüllt ist.

US 5366452 A offenbart ein Verfahren zur Herstellung eines dreidimensional gekrümmten Absoberkerns.

WO 2004011046 A1 offenbart eine absorbierendes Hafrmittel zur Herstellung von Laminaten, die in Hygieneartikeln eingesetzt werden.

JP 2002192641 A beschreibt ein Verfahren zur Herstellung eines elastischen Textilen Verbundkörpers, bei dem Fäden um einen Kern gewickelt werden.

### BESCHREIBUNG DER ERFINDUNG

Superabsorber erfahren während der Flüssigkeitsaufnahme eine Gewichtszunahme von 2500-5000%. Die damit einhergehende Volumenvergrößerung gilt es, durch geeignete Flexibilität des umgebenden Trägermaterials aufzunehmen. Im Falle klassischer Zellstoff/Superabsorber Saugkissen ist dies grundsätzlich kein Problem, da der Zellstoffausdehnung in allen drei Dimensionen ermöglicht. Im Falle sogenannter Superabsorber-Laminate, in denen Superabsorber-Granulat oder Superabsorber/ Fasermischungen mittels Haftkleber oder thermisch zwischen zwei oder mehrere Lagen Nonwovens, Folie, Tissue, o.ä. fixiert wird, müssen die äußeren Lagen des Laminats diese Funktion ermöglichen, sei es durch Dehnung oder durch geometrische Flexibilität. Dabei imuss hingegen die Einschließung des Superabsorbers jederzeit gewährleistet sein, ohne dass es zum Bruch einer der äußeren Lagen oder zum Aufreißen der Laminierung kommt. Es ist weiterhin wünschenswert, dass dieses Laminat nicht nur senkrecht zu seiner Herstellungsebene, sondern auch in dieser Ebene selbst elastisch dehnbar ist, um sowohl die Volumenvergrößerung des Superabsorbers durch die Flüssigkeitsaufnahme zu erleichtem, als auch in Kombination mit anderen Komponenten der oben bezeichneten Hygieneartikeln deren Flexibilität und Anpassungsfähigkeit an die jeweilige Körperkontur nicht zu beeinträchtigen.

Der Erfindung liegt damit die Aufgabe zugrunde, ein Laminat zur Verfügung zu stellen das eine hohe Produktionskapazität ermöglicht, sowohl verbesserte flächenelastische Eigenschaften zur optimalen Anpassung an die Körperkontur des Benutzers, als auch volumenelastische Eigenschaften zur Aufnahme großer Flüssigkeitsmengen aufweist.

### Die Aufgabe wird durch das Verfahren nach Anspruch 1 gelöst.

### In dem Zusammenhang wird ein nicht zur Erfindung gehörendes Laminat offenbart, wobei dieses Laminat ein wasserabsorbierendes Polymer (Superabsorber (SAP)) zwischen einer oder mehrerer elastischer Zwischenlagen einschließt, wobei die Zwischenlagen aus flächigen Materialbahnen bestehen die auf der Außenseite von Einzelfäden, -strängen oder Bändern fest verbunden sind und dieses Laminat im Wesentlichen quer zur Herstellungsrichtung dehnbar und im entspannten Zustand gerafft ist, und die elastischen Zwischenlagen viele einzelne Sektionen oder Kasetten von Superabsorber einschließen und der Ausdehnung des Laminats bei der Flüssigkeitsaufnahme senkrecht zur und in der Produktionsebene Raum schaffen.

Vorteilhafterweise entspricht das Aussehen der so hergestellten nebeneinander liegenden Sektionen bzw. Kasetten, der einer Steppdecke mit einer endlosen Anzahl von aneinanderliegenden Sektionen.

Vorteilhafterweise kann das Laminat für moderne ultra-dünne und elastische Hygieneartikel eingesetzt werden, da das Laminat vollständig elastisch und dehnbar ist und sich sowohl im trockenen, als auch im feuchten Zustand perfekt der Körperkontur anpasst.

Das Laminat besteht in einer Ausführungsform aus zwei äußeren Schichten, von denen eine hydrophil und die andere hydrophob ausgebildet ist.

Der Gegenstand der Erfindung ist es ein Herstellungsverfahren für das Laminat zur Verfügung zu stellen, welches die kontinuierliche Herstellung von saugfähigem Laminat mit hoher Produktionskapazität ermöglicht, wobei das Endprodukt sowohl flächenelastische Eigenschaften zur optimalen Anpassung an die Körperkontur des Benutzers, als auch volumenelastische Eigenschaften zur Aufnahme großer Flüssigkeitsmengen aufweist.

Diese Aufgabe wird durch ein Verfahren gelöst, bei dem der wasserabsorbierendes Polymer (Superabsorber (SAP)) in eine oder mehrerer elastischer Zwischenlagen eingebracht wird, wobei die Zwischenlagen aus Einzelfäden, -strängen oder Bändern zwischen zwei äußere Lagen eines so hergestellten Laminats bestehen und dieses im Wesentlichen quer zur Herstettungsrichtung dehnbar gemacht und im entspannten Zustand gerafft wird, so dass die elastischen Zwischenlagen viele einzelne Sektionen oder Kassetten von wasserabsorbierendem Polymer (Superabsorber) einschließen und der Ausdehnung des Laminats bei der Flüssigkeitsaufnahme senkrecht zur und in der Produktionsebene Raum schaffen. **Dieses Verfahren ist in Anspruch 1** definiert.

Die Raffung und Textur an der hautseitigen Oberfläche des Laminats, bzw. derjenigen Seite, von der die aufzunehmende Flüssigkeit das Laminat trifft gewährleistet vorteilhafterweise in Verbindung mit einer entsprechenden hydrophilen und transportfähigen äußeren Schicht eine ausgezeichnete Flüssigkeitsleitung sowohl in trockenem, als auch in feuchtem Zustand.

Vorteilhafterweise ist ein so hergestelltes Laminat selbst in gequollenem Zustand im Bereich der Verleimung der elastischen Lagen jederzeit senkrecht zu seiner Produktionsebene durchlässig, ohne durch die Quellung des Superabsorbers behindert zu werden, so dass die Transportleistung auch auf der Haut abgewandten Seite durch die Textur der Oberfläche und die Wahl der entsprechenden äußeren Schicht gewährleistet ist und die hautseitige äußere Schicht hinsichtlich Rücknässung bzw. der Hautfeuchte optimiert werden kann. Des Weiteren bleibt die Integrität des Laminats erhalten.

In einer weiteren Ausführung können die Außenschichten des Laminats entweder aus einem hydrophilen und einem hydrophoben flächigen Material oder beide Schichten aus hydrophilen oder hydrophoben flächigem Material bestehen. Als Materialien können beispielsweise Cellulose- oder Zellstoff verwendet werden. Die äußere Materialbahn kann aus unterschiedlichen Materialien aus der Gruppe der textilen Materialien, wie Baumwolle, Wolle, Kunststoffgamen oder anderen kunststoffhaltigen Zusammensetzungen oder ähnlichen, Spinnvliesen, Papier, Polymerfolien jeglicher Art wie Polypropylen, Polyethylen, Nylon oder ähnlichen, kardiertem Vlies, Filzstoffen oder ähnlichem bestehen

Im Fall der hydrophoben Ausführungsform erfolgt eine vorherige Mikroperforierung des flächigen hydrophoben Materials. Es wurde vorteilhafterweise gefunden, dass die Mikroperforierung eine Kanalbildung" erzeugt, die einen schnelleren Abtransport der Flüssigkeit ermöglicht und die Hautfeuchte deutlich reduziert. In einer weiteren Ausführungsform erfolgt die Kanalbildung durch eine chemische Reaktion mit dem Haftkleber der auf die Materialbahnen aufgebracht wird.

Erfindungsgemäß wird zunächst das vordere Ende der ersten äußeren textilen Materialbahn auf den Endabschnitt eines langgestreckten stab- oder rohrförmigen Formkerns aufgebracht und unter Erteilung einer Vorzugsbewegung um den Formkern herum in eine schlauchartig geschlossene Form gefaltet wird.

Im Verfahrensablauf erfolgt bei der Vorzugsbewegung auf dem Formkem durch eine Verjüngung des Querschnitts des Formkerns der Umfang dieses Schlauchs dahingehend verkürzt, dass überschüssiges Material durch außerhalb des Schlauches liegende Führungsschienen oder -stäbe in geeignete Aussparungen des Formkerns geführt wird. Anschließend wird durch mindestens eine, bevorzugt zwei und besonders bevorzugt mehr Sls zwei, den Formkern ringförmig umgebende und paarweise gegenläufige, angetriebene Zuführvorrichtungen 11 und 14 von in Umfangsrichtung beabstandeten Einzelführungen 17 Gruppen von elastischen Fäden oder Strängen 50 und 53 lose abgezogen und ohne oder nur mit reibungsbedingter geringer Vorspannung um die erste äußeren Materialbahn im Verlauf des Vorzugs auf dem Formkern aufgelegt. Auf das die den Schlauch der ersten äußeren Materialbahn kreuzförmig-symmetrisch umgebenden Flächenmuster 32 der elastischen Fäden oder Stränge wird ein Haftkleber 29 fadenförmig aufgebracht, welcher die im Wesentlichen frei liegenden elastischen Fäden benetzt und umschlingt. Im weiterem Verlauf der Vorschubbewegung wird durch Vergrößerung der Querschnitts des Formkerns der Schlauch der ersten äußeren Materialbahn gestrafft und so mit den elastischen Fäden in Kontakt gebracht und letztlich in eine flache Form gebracht. Beidseits dieses flachen Schlauches werden mittels eines Paars von Vorzugs- oder Andruckwalzen 56 zwei Einzelbahnen der zweiten äußeren Materialschichl 44 auf die freie Oberfläche der ersten äußeren Materialbahn 20 und der mit dem Haftkleber 29 versehenen elastischen Fäden 17 verbunden. Während dieser Verbindung werden beidseits des Schlauchs der ersten äußeren Materialbahn zwischen dieser und der zugeführten zweiten äußeren Materialbahn einzelne Spuren von Superabsorber 47 eingebracht. Abschließend wird der so erzeugte Schlauch längs in Einzelbahnen aufgetrennt und diese traversierend auf Einzelrollen gewickelt oder in Kisten abgelegt.

Durch die Einfaltung des Schlauches der ersten Materialbahn und Erzeugung der Umfangselastizität des Schlauches durch Aufspreizung der ersten äußeren Materialbahn unter Ausdehnung des elastischen Fadenmusters 32 ergibt sich gegenüber vergleichbaren aus dem Stand der Technik bekannten Wickelverfahren für elastische Schläuche auf der Basis von vorgespannten Fäden oder Bändern wie sie z.B. aus der WO 03041627 oder DE 102004026070, vorteilhafterweise eine erhebliche Reduzierung der Komplexität durch Eliminierung des Antriebs der Einzelführungen der Fäden und eine proportional zur Querdehnbarkeit des Laminats erfolgende Erhöhung der Produktionsmenge. Dies beruht auf der Grenzdrehzahl die die Produktionsgeschwindigkeit der ringförmigen Zuführvorrichtungen begrenzt. Des Weiteren wird die erfindungsgemäß die Breite des Auftragssystems für den Haftkleber 29 in selbem Maße vorteilhafterweise reduziert.

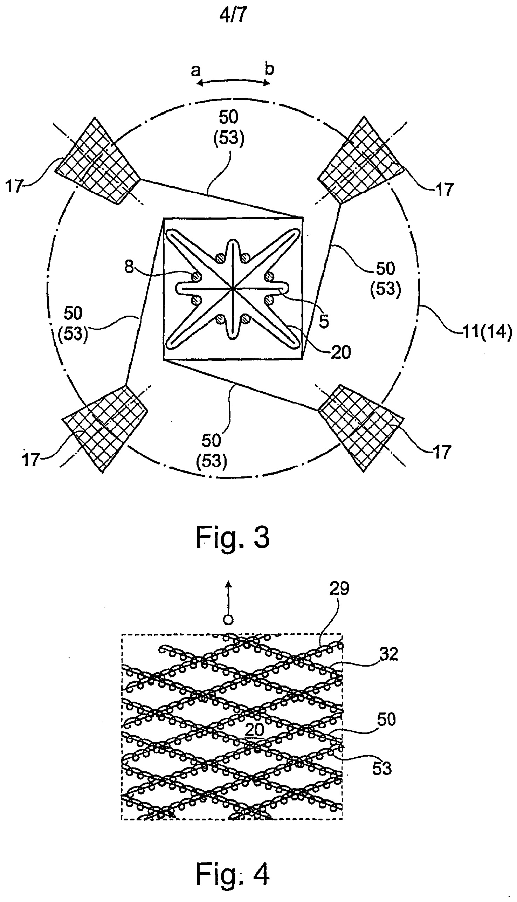

)Da nach Aufbringung der elastischen Fäden oder Bänder auf den Schlauch der ersten äußeren Materialbahn dieser nur punktuell berührt wird und ansonsten frei liegt, wird ein spiral- oder meanderförmig aufgebrachter Haftkleber durch das Flächenmuster der elastischen Fäden gezwungen. Bevorzugt wird dies, um diese elastischen Fäden oder Bänder zu umschlingen und nach Aufspreizung des Schlauches der ersten äußeren Materialbahn sich im Wesentlichen um die Fäden zu orientieren. Hierdurch wird ein dem Flächenmuster 32 entsprechendes Leimbild wie, z.B. in der Fig. 4 erzeugt welches aufgrund der Umschlingung der elastischen Fäden dieser sowohl mit der ersten, als auch mit der zweiten äußeren Materialbahn im weiteren Verlauf der Vorzugsbewegung auf dem Formkern haftend verbindet.

Die äußere Materialbahn kann aus unterschiedlichen Materialien aus der Gruppe der textilen Materialien, wie Baumwolle, Wolle, Kunststoffgamen oder anderen kunststoffhaltigen Zusammensetzungen oder ähnlichen, Spinnvliesen, Papier, Polymerfolien jeglicher Art wie Polypropylen, Polyethylen, Nylon oder ähnlichen, kardiertem Vlies, Filzstoffen oder ähnlichem bestehen.

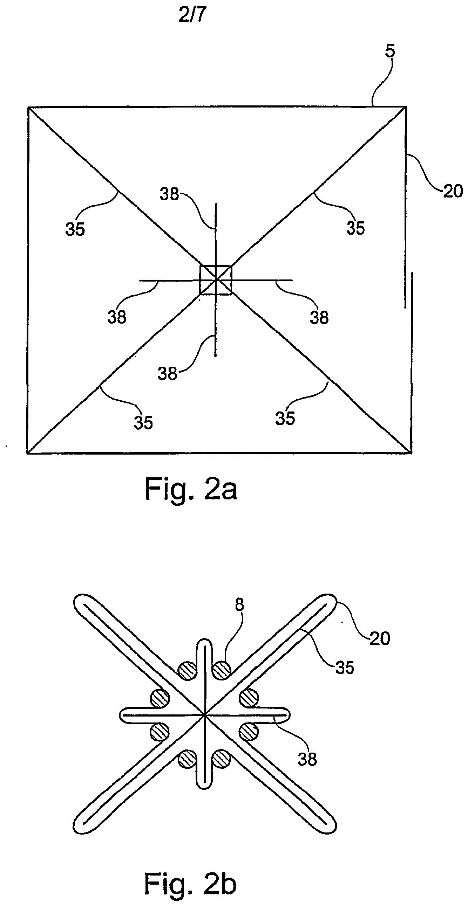

In einer weiteren Ausführung besteht, den Formkern 5, wie z.B. in der Fig. 2a gezeigt aus Führungsstreben 35, welche die Materialbahn 20 führen. Dazwischen sind Speicherstreben 38 angeordnet (Fig. 2a), in welche während der Verjüngung des Formkerns durch entsprechende Führungsschienen 8 überschüssiges Material des Schlauches der Materialbahn 20 eingedrückt wird (Fig. 2b).

Vorteilhafterweise kann die einmal in die Speicherstreben eingedrückt, erste äußere Materialbahn leicht durch Unterdruck in einer an die Führungs- und Speicherstreben des Formkerns eng anliegenden Position gehalten werden und reibungsarm über den Formkern gezogen werden. Als weitere Alternativen zur Fixierung können die Einbringung von Pressluft oder durch elektrostatische Aufladung genannt werden, die ebenfalls zu der eng anliegenden Position führen und gehalten werden und reibungsarm über den Formkern gezogen werden.

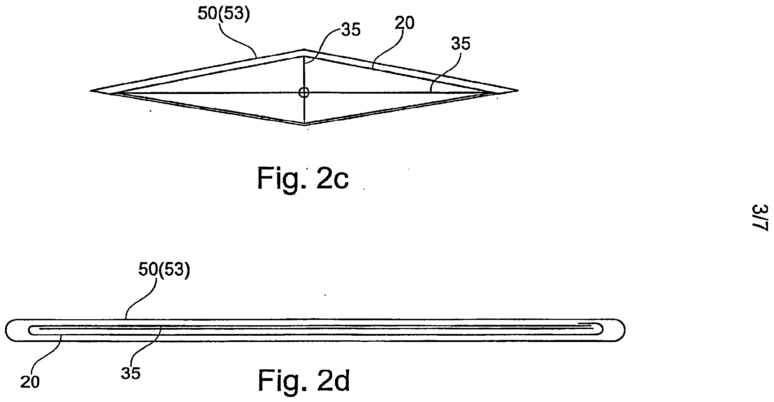

Die Aufspreitzung des Formkerns nach Aufbringung der elastischen Zwischenlagen und des Haftklebers wird vorteilhafterweise dadurch gelöst, dass paarweise gegenüberliegende Führungsstreben im weiteren Verlauf des Formkerns in ihrer Ausdehnungsrichtung quer zur Längsachse des Formkerns sukzessive verbreitert, ein weiteres Paar Führungsstreben in selbem Maße in seiner Breite verringert wird, so dass der Schlauch der ersten äußeren Schicht kontinuierlich aus den Speicherstreben gezogen und über die Spitzen der )Führungsstreben straff gezogen und so mit den haftverleimten elastischen Lagen in flächigen Kontakt gebracht wird (Fig.2c), letztlich im weiteren Verlauf der Vorzugsbewegung durch weitere synchrone paarweise Verbreiterung, respektive Breitenreduzierung der Führungsstreben bei im Wesentlichen gleicher Umfangslänge in einen flachen Zustand gebracht wird (Fig. 2d).

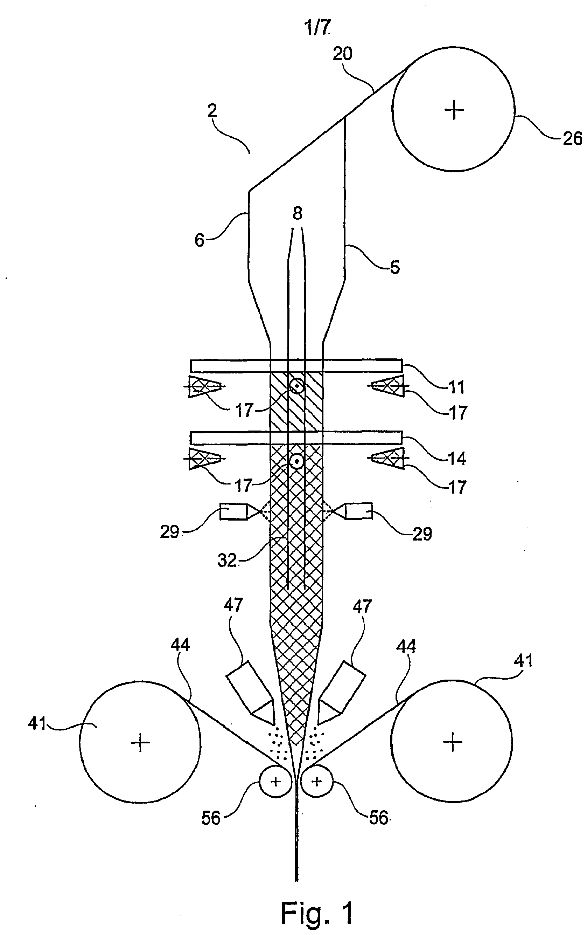

Vorteilhafterweise wird der Formkern 5 mit seiner Längsrichtung senkrecht angeordnet, dass die Vorzugsrollen 56 sich am unteren Ende des Formkerns 5 befinden (Fig.1).

Die Zufuhr des wasserabsorbierenden Polymers (Superabsorbers) ertolgt ertindungsgemäß in individuellen volumetrisch oder gewichtsdosierten kontinuierlichen Einzelbahnen oder - spuren erfolgen. Das wasserabsorbierenden Polymers (Superabsorbers) kann entweder intermittierend durch pulsierende Druckluft oder Kolben in die leimfreien Bereiche des Flächenmusters eingetragen oder intermittierend dem Flächenmuster folgend quer zur Fliehrichtung des Schlauchs der ersten äußeren Materialbahn ausgelenkt werden. Für einfache Anwendung kann es hinreichend sein, diese Einzelspuren kontinuierlich einrieseln zu lassen, wobei zweckmäßigerweise pro Kassette des Flächenmusters zwei Einzelbahnen zugeführt werden.

Erfindungsgemäß können die die Vorzugsrollen sektionsweise mit Unterdruckbereichen versehen werden, so dass die Ablage des wasserabsorbierenden Polymers (Superabsorbers) auf der leimfreien Sektion bzw. Kassetten des Flächenmuster präzise erfolgt, so dass kein wasserabsorbierenden Polymers (Superabsorbers) in die angrenzenden Bereich entweicht.

In einer weiteren Ausführungsform kann der Schlauch der ersten äußeren Materialbahn 20 vor der Zufuhr in die Vorzugrollen 56 in Querrichtung durch Verringerung der Breite der Führungsstreben entspannt werden, so dass diese bei der Zuführung der zweiten äußeren Bahn 44 eine leichte Welligkeit aufweist. Dies begünstigt die die Bildung von Taschen im Bereich der nicht verleimten Zonen des Flächenmusters die die Ablage des 32 Superabsorbers 47 erleichtern.

In einer weiteren Ausführungsform der Erfindung kann durch Kontrolle dieser Welligkeit das Verhältnis der Materialbreiten der ersten und zweiten äußeren Materialbahnen zueinander vorteilhafterweise so gesteuert werden, dass das erzeugte Laminat auf der Seite der ersten äußeren Schicht eine höhere Welligkeit gegenüber der Seite der zweiten äußeren Schicht aufweist. Hierdurch ist bei gleichem Quellvolumen der einzelnen Kassetten eine Minimierung des jeweils teuren Materials der äußeren Schichten 20 und 44 möglich.

### BESCHREIBUNG DER ZEICHNUNGEN

Anhand der nachfolgenden Zeichnungen soll die vorliegende Erfindung näher erläutert werden ohne sie auf diese Ausführungen zu beschränken.

Es zeigen
Fig.1 stark schematisierte Darstellung der Seitenansicht der wesentlichen Funktionselemente einer Ausführungsform des erfindungsgemäßen Verfahrens.
Fig. 2a Aufbau des Formkerns aus Führungs- und Speicherstreben;
Fig. 2b Aufbau des Formkerns aus Führungs- und Speicherstreben und Führungsschienen im Bereich der Verjüngung des Formkerns;
Fig. 2c Aufbau des Formkerns im Bereich der Aufspreizung des Formkerns;
Fig. 2d Aufbau des Formkerns nach vollendeter Aufspreizung ;
Fig. 3 Anordnung von Einzelführungen in einer der in der Vorrichtung gemäß den Figuren 1 und 2 zur Aufbringung von Gruppen von ungespannten oder leicht gespannten Fäden oder Strängen vorgesehenen Zuführeinrichtungen,
Fig.4 bei der Verwendung von zwei gegensinnig angetriebenen Zuführeinrichtungen gemäß Fig. 3 erhaltene Flächenmuster der elastischen Fäden oder Stränge und des aufgebrachten Haftklebers in einem in der erfindungsgemäßen Weise hergestellten Laminats.
Fig. 5a bei der Verwendung von zwei gegensinnig angetriebenen Zuführeinrichtungen gemäß Fig. 3 erhaltenes Flächenmuster der elastischen Fäden oder Stränge und des punktuell zugeführten pulverförmigen Füllmaterials in dem erfindungsgemäß hergestellten Laminat,
Fig. 5b bei der Verwendung von zwei gegensinnig angetriebenen Zuführeinrichtungen gemäß Fig. 3 erhaltenes Flächenmuster der elastischen Fäden oder Stränge und des kontinuierlich in diskreten Einzelbahnen zugeführten pulverförmigen Füllmaterials in einem in der erfindungsgemäßen Weise hergestellten Laminats.
Fig. 5c bei der Verwendung von zwei gegensinnig angetriebenen Zuführeinrichtungen gemäß Fig. 3 erhaltene Flächenmuster der elastischen Fäden oder Stränge und des kontinuierlich in diskreten Einzelbahnen zugeführten, quer zur Produktionsrichtung intermittierend ausgelenkten pulverförmigen Füllmaterials in einem in der erfindungsgemäßen Weise hergestellten Laminats,
Fig.6 schematische Darstellung des erfindungsgemäß hergestellten Laminats bei Entlastung des Schlauches der ersten Materialbahn und der eingelagerten elastischen Zwischenlagen quer zu deren Produktionsrichtung vor Einbringung des Superabsorbers und der Verbindung mit der zweiten Äußeren Matereialbahn.
Fig. 7a schematische Darstellung eines Ausschnitts des Querschnitts des Formkerns bei Verwendung von zwei Speicherstreben zwischen jeweils zwei Führungsstreben und der Kontrolle des sektionsweisen Einzugs der ersten äußeren Schicht durch Positionierung der Führungsschienen.
Fig. 7b Flächenmuster der elastischen Fäden des nach Fig. 7a hergestellten elastischen Laminats in ebenen, entspannten Zustand.

Die Fig. 1 zeigt vereinfacht und stark schematisiert dargestellt die Vorrichtung 2 zur Herstellung des Laminats. Die Vorrichtung besteht in dieser Ausführungsform aus einem langgestreckten Formkern 5 mit einem quadratischem Querschnitt, der durch zwei in Längsrichtung voneinander beabstandeten Zuführeinrichtungen 11 und 14 umgeben ist. Diese Zuführeinrichtungen sind im Sinne der durch die Pfeile a und b veranschaulichten Richtungen gegensinnig drehangetrieben. Auf diesen ringförmigen Zuführeinrichtungen sind jeweils in Umfangsrichtung verteilt voneinander beabstandete Einzelführungen 17 für elastische Fäden, Bänder oder Stränge angeordnet. Über diese Einzelführungen werden die elastischen Elemente in axialer Richtung von ihrer Aufwicklung abgezogen und in Richtung zum Formkern 5 geführt.

Die Figur 2a zeigt eine drehbar gelagerte und angetriebene Materialrolle 26, von der eine erste äußere Materialbahn 20 in der vorliegenden Ausführungsvariante bahnspannungsgesteuert gefördert wird, und über eine Formschulter 6 in einen Schlauch rechteckigen Querschnitts gefaltet und auf das vordere Ende durch mehrere Streben 35, 38 gebildeten Formkerns 5 aufgelegt wird. Vorteilhafterweise wird hierdurch die Überlappung der ersten äußeren Materialbahn 20 bei der Bildung des Schlauches durch Aufbringung eines Haftklebers, Verschweißen oder mechanische Verhaftung fixiert.

Die Figur 2b wird der so gebildete Schlauch anschließend über den sich in seinem Querschnitt verjüngenden Formkern geführt, so dass dieser den Formkern lediglich auf den Enden der sich in ihrer Breite verkürzenden Führungsstreben 35 geführt wird und dazwischenliegendes Material des Schlauches mittels Führungsschienen oder -drähten 8 in durch zwischen den Führungsstreben angeordneten Speicherstreben 38 geformte Freiräume geführt wird .

In der Figur 3 wird dieser so in seinem Umfang eingekürzte Schlauch anschließend durch zwei gegenläufig drehende Zuführeinrichtungen 11 und 14 geführt. In den Zuführeinrichtungen werden von jeweils in Umfangsrichtung beabstandeten Einzelführungen 17 stationärer Spulen oder Rollen einzelne elastische Fäden, Bänder oder Stränge 50,53 abgezogen und auf den Schlauch der ersten äußeren Materialschicht 20 aufgelegt. Durch die Überlagerung der Drehbewegungen der beiden Zuführeinrichtungen 11,14 mit dem Vorzug der ersten Materialschicht 20 ergibt sich ein gegenläufig diagonales Muster der elastischen Elemente 50, 53, welches mit der ersten Materialschicht lediglich auf den Spitzen der Führungsschienen 35 des Formkerns 5 in Kontakt ist, ansonsten aber frei liegt, Fig. 3.

Im weiteren Verlauf des Vorzugs auf dem Formkern 5 wird auf die elastischen Fäden 50 und 53 Haftkleber bevorzugt als ein Vorhang von Spiral- oder Mäanderfäden aufgetragen, welcher bei Aufschlag auf das freiliegende gegenläufig-diagonale Muster der elastischen Fäden 50 und 53 von diesen umgelenkt und bevorzugt die elastischen Fäden, speziell aber die Kreuzungspunkte der Fäden 50 und 53 zueinander benetzt und umfängt, Fig. 4.

Im weiteren Verlauf des Vorzugs auf dem Formkern 5 wird dieser in seinem Querschnitt derart gespreizt, dass jeweils zwei gegenüberliegende Führungsstreben in ihrer Breite dahingehend erweitert und ein komplementäres Paar von Führungsstreben in seiner Breite dahingehend verringert wird, das zunächst das in den Speicherstreben 38 des Formkerns 5 verbliebene Material der ersten Schicht 20 kontinuierlich gelöst wird und der Schlauch der ersten Materialschicht 20 über die Enden der Führungsschienen 35 straff gespannt wird (Fig. 2c). Hierdurch wird der Schlauch der ersten Materialbahn 20 flächig in Kontakt mit dem Muster der elastischen Elemente 17 und des auf diese aufgebrachten Haftklebers gebracht. Der Prozess der paarweisen Erweiterung der Führungsstreben 35 und der einhergehenden Verkleinerung komplementärer Führungsstreben wird im Verlauf des weiteren Vorzugs des Schlauchs der ersten Materialbahn 20 über den Formkern 5 dahingehend fortgeführt, das letztlich der Schlauch der ersten Materialbahn 20 nur über die Enden von zwei Führungsstreben 35 des Formkerns 5 geführt wird und damit praktisch eben ist.

In einem weiteren Verfahrensschritt wird dieser so geformte und in eine flache Form gebrachte Schlauch in eine Vorrichtung von zwei angetriebenen gegenläufigen Vorzugselementen, in der vorliegenden Ausführungsvariante in Form zweier Rollen oder Walzen 56 eingeführt, welche den Vorzug für das Abrollen der ersten äußeren Schicht 20 von der Materialrolle 26 und den Transport aller Materialien über den Formkern liefert.

Über die Vorzugswalzen 56 wird beiderseits der Materialbahn 20 von zwei von einander beabstandeten, drehend angetriebenen Materialrollen 41 je eine Einzelbahn der zweiten Äußeren Schicht 44 vorzugsweise bahnspannungskontrolliert abgezogen und zugeführt und mit der ersten Materialschicht 20 und dem elastischen Flächenmuster 32 flächig über den Haftkleber 29 verbunden.

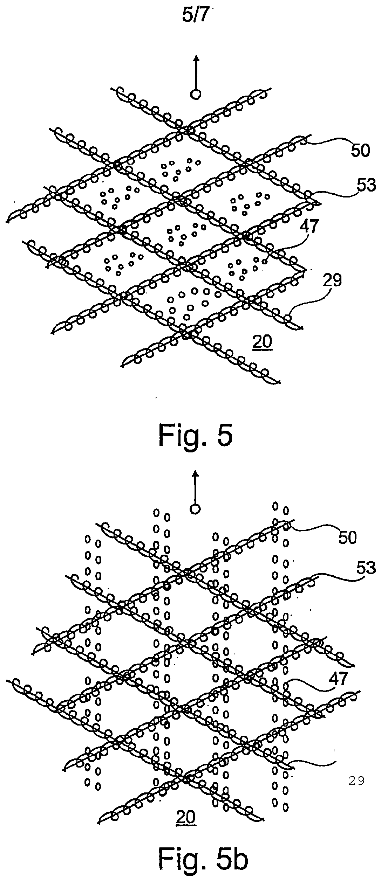

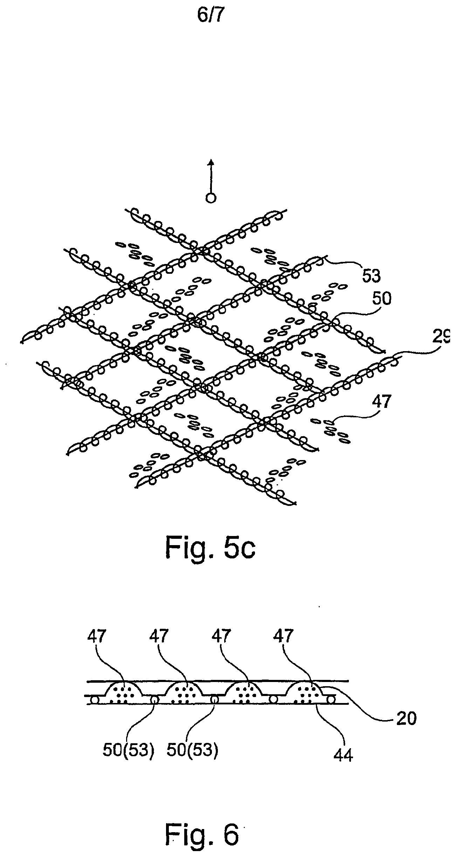

Bei der Zusammenführung des abgeflachtem Schlauchs der ersten Materialbahn 20 mit den zugeführten ebenen Bahnen der zweiten Materialbahn 44 durch die Vorzugseinheit 56 wird beidseitig des Schlauches senkrecht ein wasserabsorbierendes Polymer (Superabsorber 47) derart eingebracht, das entsprechend auf das Flächenmuster 32 abgestimmte, einzeln volumetrisch oder grammetrisch auf die Vorzugsgeschwindigkeit des Schlauches der ersten Materialbahn 20 abgestimmte Einzelbahnen oder -spuren kontinuierlich eingerieselt werden, wodurch sich ein Produktmuster gemäß Fig. 5b ergibt. In einer alternativen hier nicht dargestellten Variante können diese Spuren auch intermittierend oder oszillierend quer zur Fließrichtung entsprechend dem Flächenmuster 32 mechanisch oder pneumatisch abgelenkt (Produktmuster gemäß Fig. 5c) oder diese Spuren entweder durch pulsierende oder intermittierende Druckluft, mechanische Kolben oder mechanische Auslenkung der Fördereinrichtung quer zu Fließrichtung des Schlauches der ersten Materialbahn 20 auf die Zentren der durch das Flächenmuster 32 gebildeten haftkleberfreien Bereiche dieses Schlauches gelenkt werden wie z.B. gemäß dem Produktmuster der Fig. 5a. In einer weiteren hier nicht dargestellten Variante erfolgt das gezielte Auftragen des wasserabsorbierendes Polymer (Superabsorber 47) über die Vorzugsrollen, die sektionsweise mit Unterdruckbereichen versehen werden, so dass die Ablage des wasserabsorbierenden Polymers (Superabsorbers) auf der leimfreien Sektion bzw. Kassetten des Flächenmuster präzise erfolgt, so dass kein wasserabsorbierenden Polymers (Superabsorbers) in die angrenzenden Bereich entweicht.

In einer Ausführungsvariante erfolgt das Abbinden des Haftkleber durch den Andruck der Vorzugsrollen 56 nach dem Verbinden der beiden äußeren Schichten 20 und 44 miteinander, wobei zwischen den eingelagerten elastischen Elementen 50,53 die Einlagerung des wasserabsorbierendes Polymer (Superabsorber 47) erfolgt wird im Verlauf der weiteren Vorzugsbewegung dieses so geformten mehrlagigen Schlauchs längs in einzelne Bahnen aufgetrennt wird. Diese Bahnen können wahlweise traversierend auf Rollen aufgewickelt oder in Kisten abgelegt oder aufgeschossen werden.

In der Figur 6 ist eine weitere Ausführungsform dargestellt. Hier wird die Breite des flachen Schlauches aus einer ersten Materialbahn 20 und dem Flächenmuster der elastischen Elemente 32 nach dem Aufspreizen in den flachen Zustand wie in Fig. 2d durch Verringerung der Breite der Führungsschienen 35 derart entspannt und gerafft, so dass sich nach dem Verbinden mit der ansonsten im Wesentlichen flachen zweiten äußeren Materialbahn 44 ein elastisches, saugfähiges Laminat ergibt. Dieses Laminat, weist durch die erste äußere Materialbahn gebildeten Außenseite eine höhere Raffung auf als auf der durch die zweite äußere Schicht geformten Seite. )

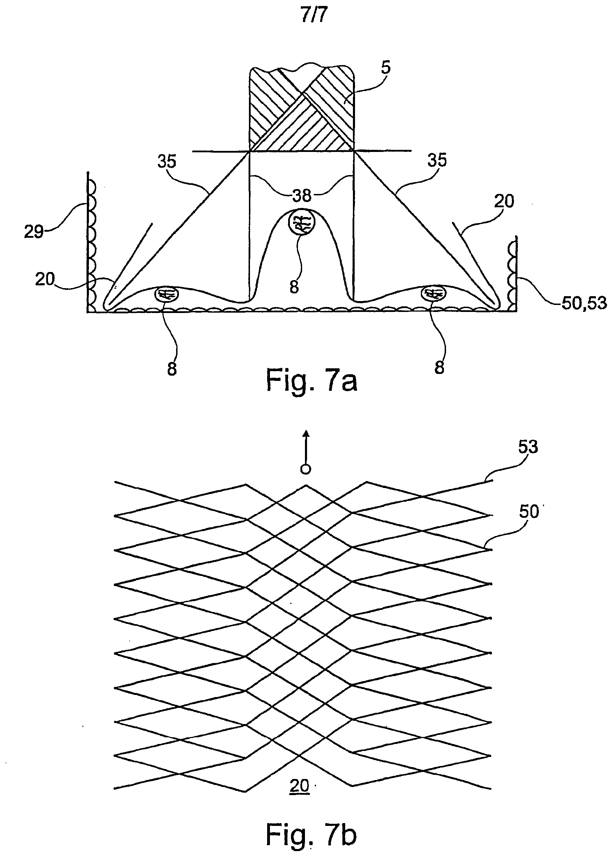

In einer weiteren Ausführungsform kann das ebene Laminat quer zu seiner Herstellungsrichtung mit Sektionen bzw. Kassetten unterschiedlicher Raffung der äußeren Lagen und somit unterschiedlichen Quellvolumens auszustatten, was durch Steuerung der Superabsorberverteilung quer zur Herstellungsrichtung des Laminats zu einer vorteilhaften Querverteilung der Absorptionsfähigkeit führt. Die Fig. 7a zeigt, zwei Speicherstreben 38 pro Führungsstrebenpaar 35, die durch Anordnung der Führungsschienen 8 in den ausgeformten Speichersektionen des Formkerns 5 in Querrichtung zur Vorzugsrichtung unterschiedliche Bahnbreiten des durch die Verjüngung des Formkerns 5 überschüssigen Bahnmaterials der ersten äußeren Materialbahn 20 eingefügt werden. Im weiteren Verlauf )der Vorschubsbewegung und Aufspreitzung des Formkerns 5 wird zunächst durch die Verbreiterung der Speicherstreben 38 auf deren Spitzen die Materialbahn 20 mit dem Haftkleber 29 der elastischen Lagen 50,53 in Kontakt gebracht und anschließend die Aufspreizung des Formkerns 5 im Sinne der Fig. 2b-d fortgeführt und die Kombination mit dem Superabsorber und der zweiten äußeren Materialbahn 44 vollendet, so entsteht letztlich ein längs aufgetrenntes Laminat, welches in entspanntem, ebenem Zustand eine in Querrichtung streifenweise Variation der Raffung der äußeren Schichten und damit letztlich der Absorptionsfähigkeit aufweist, Fig. 7b.

Es kann zweckmäßig sein, die Funktion des Eindrückens der ersten äußeren Materialbahn in die Freiräume der Speicherstreben anstelle der Führungsschienen 8 durch Unterdruck aus dem Führungskern, durch statische Aufladung der ersten äußeren Materialbahn 20 gegenüber den Streben 35,38 des Formkerns oder durch Aufbringung von Druckluft auf die Außenseite des Schlauches zu erreichen.

Es kann ferner sinnvoll und zweckmäßig sein, die Funktionen der jeweils 4 Führungs- und Speicherstreben aus Fig. 2a und 2b durch eine entsprechend höhere Zahl an Streben zu erreichen, um speziell bei großen Bahnbreiten der ersten äußeren Materialbahn 20 oder bei hohem Speicherbedarf bei der Verjüngung der Formkerne 5 eine gute Materialtührung zu erreichen.

Auch wenn die Isolierung einzelner Sektionen von Superabsorbern in leimfreien Bereichen eines ansonsten untereinander verleimten Bahnmaterials aus zwei äußeren Materialbahnen 20 und 44 und eingelagerten elastischen Lagen 50 und 53 für die Quellwirkung und Volumenvergrößerung des Superabsorbers 47 bei der verwendungsgemäßen Flüssigkeitsaufnahme zweckmäßig ist, kann es sinnvoll sein, die zweite äußere Materialbahn 44 vor Kombination mit der ersten äußeren Materialbahn 20 flächig oder in Längsstreifen mit einer zusätzlichen leichten Schicht Haftkleber zu benetzen, um den Superabsorber möglichst flächig zu fixieren, dadurch einem möglichen Verklumpen dieses Materials vor Beginn der Flüssigkeitsaufnahme vorzubeugen und die Haptik des so hergestellten Laminats zu verbessern.

### BEZEICHNUNGEN DER ELEMENTE DER ZEICHNUNGEN

- 2: Gesamtvorrichtung
- 5: Formkern
- 6: Formschulter der ersten äußere Materialbahn
- 8: Führungen erste äußere Materialbahn
- 11: Erste Zuführeinrichtung
- 14: Zweite Zuführeinrichtung
- 17: Einzelführung elastische Fäden
- 20: Erste Schicht der äußeren Materialbahn
- 23: Formschulter
- 26: Materialrolle der ersten äußere Materialbahn
- 29: Haftkleber
- 32: Flächenmuster der elastischen Fäden
- 35: Führungsstreben des Formkerns
- 38: Speicherstreben des Formkerns
- 41: Materialrolle der zweiten äußere Materialbahn
- 44: Zweite äußere Materialbahn
- 47: wasserabsorbierendes Polymer(Superabsorber)
- 50: Einzelfaden der ersten elastischen Zwischenlage
- 53: Einzelfaden der zweiten elastischen Zwischenlage
- 56: Zugrollen

## Patentansprüche

1. Verfahren zur Herstellung eines hochflexiblen, absorbierenden Laminats, bestehend aus zwei äußeren Schichten (20, 44), zwischen denen zwei unter Vorspannung stehende elastische Zwischenlagen (50, 53) und Sektionen von superabsorbierendem Granulat (47) eingebracht sind, bei welchem Verfahren eine äußere Materialbahn (20) über einen langgestreckten Formkern (55) zu einem Schlauch gefaltet, dieser durch Verjüngung des Querschnitts des Formkerns (55) verkleinert und mit Gruppen weitgehend ungespannter elastischer Fäden oder Bänder (50, 53) gegenläufig im Sinne einer Schlaucharmierung umwickelt, auf diese Gruppen elastischer Elemente Haftkleber (29) aufgebracht und diese durch Spreizung über den Formkern (55) unter Spannung mit dem Schlauch der ersten äußeren Materialbahn (20) in Kontakt, beide zusammen im weiteren Verlauf der Vorschubbewegung des Schlauches auf dem Formkern (55) in eine flache Form gebracht und unter intermittierender oder kontinuierlicher Zufuhr von Einzelspuren von Superabsorber (47) mit zwei Bahnen einer zweiten äußeren Materialbahn (44) verbunden und längs zu querelastischen Einzelbahnen aufgeschnitten wird, wobei die äußere Materialbahn (20, 44) aus textilen Materialen, wie insbesondere Baumwolle, Wolle, Kunststoffgarnen oder anderen kunststoffhaltigen Zusammensetzungen, Spinnvliesen, Papier, Polymerfolien, wie insbesondere Polypropylen, Polyethylen, oder Nylon, kardiertem Vlies oder Filzstoffen besteht.

## Claims

1. Method for producing a highly flexible, absorbent laminate consisting of two outer layers (20, 44) wherebetween are incorporated two pre-tensioned elastic interplies (50, 53) and sections of superabsorbent granulate (47), said method comprising an outer length of material (20) being folded over an elongate core (55) to form a hose, this hose being reduced in size by tapering the cross section of the core (55) and being wrapped with groups of substantially untensioned elastic threads or bands (50, 53) in a contrarotating manner in the form of a hose reinforcement, pressure-sensitive adhesive (29) being applied to these groups of elastic elements and these by spreading over the core (55) under tension being brought into contact with the hose of the first outer length of material (20), both together in the further course of the forward feed movement of the hose on the core (55) being brought into a flat shape and by intermittent or continuous supply of individual tracks of superabsorbent (47) being bonded to two lengths of a second outer length of material (44) and being longitudinally cut open to form transversely elastic individual lengths, wherein the outer length of material (20, 44) consists of textile materials, such as especially cotton, wool, plastics yarns or other plastics-containing compositions, spunbonded webs, paper, polymer films such as especially polypropylene, polyethylene or nylon, card web or felts.

## Revendications

1. Procédé de fabrication d'un stratifié absorbant hautement flexible comprenant deux couches extérieures (20, 44) entre lesquelles se trouvent deux nappes intermédiaires élastiques (50, 53), placées sous précontrainte, et des sections de granulés super-absorbants (47), procédé dans lequel une bande de matériau extérieure (20) est repliée sur un mandrin allongé (55) pour former un tuyau, ce dernier est réduit par amincissement de la section transversale du mandrin (55) et enroulé dans des sens opposés avec des groupes de fils ou bandes élastiques (50, 53) dans une large mesure non tendus dans le sens d'un renfort de tuyau, un adhésif (29) est appliqué sur ces groupes d'éléments élastiques et ceux-ci sont mis en contact sous tension avec le tuyau de la première bande de matériau extérieure (20) par étalement sur le mandrin (55) tout en faisant avancer le tuyau sur le mandrin (55) et est relié à deux bandes d'une deuxième bande de matériau extérieure (44) par apport intermittent ou continu des pistes individuelles de super-absorbant (47) et coupé longitudinalement aux bandes individuelles élastiques transversalement, la bande de matériau externe (20, 44) comprenant des matières textiles, tels que du coton, de la laine, des fils en matière synthétique ou d'autres compositions contenant de matière synthétique, des non tissés, du papier, des films de polymères, tels que notamment du polypropylène, du polyéthylène ou du nylon, un non-tissé cardé ou des matériaux en feutre.
